# EUROPEAN PATENT SPECIFICATION

(11) **EP 1 670 386 B1**
(45) Date of publication and mention of the grant of the patent: **22.04.2009**
(21) Application number: 03818686.2
(22) Date of filing: 22.11.2003
(51) Int. Cl.: A61F 2/06

(54) **LINED BALLOON MOUNTED STENT**
AUSGEKLEIDETER BALLONMONTIERTER STENT
EXTENSEUR MONTE SUR BALLONNET DOUBLE

(30) Priority: 24.09.2003 EG 20030947
(43) Date of publication of application: 21.06.2006
(73) Proprietor: Lotfy, Wael Mohamed Nabil, Cairo 12211 (EG)
(72) Inventor: Lotfy, Wael Mohamed Nabil, Cairo 12211 (EG)
(74) Representative: Freischem, Stephan
(86) International application number: PCT/EG2003/000009
(87) International publication number: WO 2005/027791

(56) References cited:
- WO-A1-01/52769
- US-A- 5 234 456
- US-A- 5 370 691
- US-A- 6 007 575
- US-B1- 6 293 968

## Description

The present invention is directed to the field of intravascular stents.

The document US 6 293 968 B1 describes an inflatable intraluminal vascular stent which incorporates fine conduits of synthetic material in a meshwork like structure forming a tubular vascular prosthesis. The conduits provide for inflating and deflating the meshwork structure to deploy the stent, adjust its supporting force and for repositioning or removing the stent, if needed.

Following insertion of the stent into the treatment zone, the meshwork structure is inflated to expand the stent against the vasculature wall. Importantly, the inflation pressure is controllable to regulate the supporting force of the stent as it conforms to the shape of the vasculature. The stent is combined with a graft. The graft is a hollow tube of prosthetic material such as Dacron.

### Lined Balloon Mounted Stent (detailed description):

This invention is about to mount a non reactive inflatable tissue on an appropriate size intravascular stent to control the flow distally. This created stent is then placed on the intended vessel per catheter. This tissue can be put in different designs:
1. A tire in a wheel with a central opening.
2. In the form of successive openings of different sizes to allow for future change in the size of the stenosis imposed to the circulation by interventions e.g. balloon catheters to remove one of the narrowings for example.
3. Crescentic or boggy masses of enclosed tissue that can be compressed later on e.g. by a balloon to modify the gradient across the stenosis produced e.g. pulmonary artery.
4. A design similar to naturally occurring stenotic valves.
5. A stenotic absorbable material to allow for natural progressive dilatation.
6. A stenotic material that swells with time to allow for progressive narrowing.

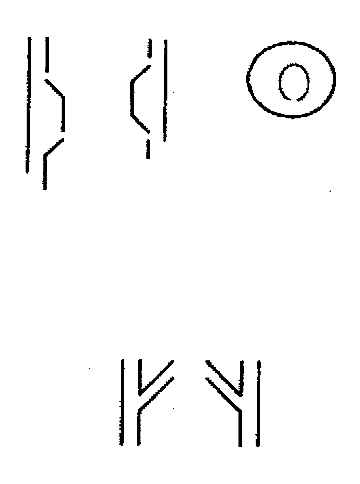

Figure 1 and 2 demonstrate a sketch of one version of items 1 and 4.

All these designs can be inflated or deflated to control their size during the procedure and sometimes later as well. The inflation can be done by carbon dioxide, air or even different fluids e.g. normal saline. The addition of the ability to compress the narrowed segment later on by dilating balloons is again feasible as well.

This could replace state of the art procedures e.g. the pulmonary artery band that we know and are using now.

For this purpose the metallic dilatable stents in common use in cardiology practice can be prepared to hold the balloon inside it. The balloon material that can be used is similar to the one used in valvotomy balloons in our current practice, however the essential requirement is only inflatability and non reactivity.

As this procedure is expected to be done in the catheterization laboratory, I believe it would be executed with much less mortality, morbidity and expense as compared to its surgical counterpart. I expect it thus to revolutionize the practice. Because the ability to perform a per catheter band without mortality will definitely make surgical corrections of some simple as well complicated cardiac lesions not needed or at least deferrable to the time where they could be done with less mortality. If we combine this by the ability to control the pressure gradient during insertion (e.g. doing echo or direct measurement in the cath and ascertaining the hemodynamic consequences directly). Again, the ability to reduce or increase the pressure gradient at the same setting or at later settings. For more complex lesion, it can be done as a permanent palliation or in preparation for future palliation.
I suggest the name of Lotfy's stent for the stent that will be designed for this purpose.

### The previous state of the art:

To achieve control over the blood flow inside the vessels a surgical procedure is undertaken (with its inherent costs, risks) to band the vessel from outside. Different systems had been devised for this but they were all applied from outside the vessel.

Problems in the previous state of the art:
1. High cost of the surgery, with inherent risks and problems associated with the throracic surgeries.
2. Sometimes the condition of the patient (e.g. a sick baby) is not suitable for the operation despite its urgency.
3. The inability to change the degree of the band once the operation is over except with another operation with again higher risks.
4. Fibrosis and distortion produced during and after the surgery would make future operations in the area involved more difficult.

### What is new about the invention ?

1. Achieve the same result as the surgical intervention.
2. Avoid the risk and complications of surgery and reoperation.
3. The ability to change the degree of narrowing produced during and after the catheter procedure.

The procedure of pulmonary artery banding and related procedures was never reported in the literature to be done intravascularly.

### How can it be used ?

A selected company producing the common use intravascular stent will be chosen after agreement with the inventor to upgrade some of its stents with the new designs and linings I suggested.

## Claims

1. Flow control or flow limitation device comprising:
■ a stent to be placed inside a vessel, and
■ a lining made of non-reactive material fixed inside said stent, **characterized in that** said lining is inflatable and deflatable to control the flow through.

2. Device of claim 1, **characterized in that** the stent is at least one of the following:
■ made of metallic material;
■ made of plastic material;
■ totally inflatable;
■ shaped as a ring;
■ of tubular shape;
■ of cylindrical shape;
■ of conical shape;
■ of pentagonal shape.

3. Device of one of the preceding claims, **characterized in that** the lining is made of PTFE.

4. Device of one of the preceding claims, **characterized in that** the stent is adapted to be placed inside
■ a blood vessel or
■ airways or
■ urinary passages or
■ gastrointestinal passages or
■ industrial pipes.

## Patentansprüche

1. Flussregelungs- oder Flussbegrenzungsvorrichtung, umfassend:
• einen innerhalb eines Gefäßes zu platzierenden Stent und
• eine aus nicht reaktivem Material hergestellte, innerhalb des genannten Stents befestigte Auskleidung,
**dadurch gekennzeichnet, dass** man die genannte Auskleidung aufblasen und die Luft aus ihr entfernen kann, um den Durchfluss zu regeln.

2. Vorrichtung nach Anspruch 1, **dadurch gekennzeichnet, dass** der Stent mindestens eine der folgenden Eigenschaften aufweist:
• hergestellt aus metallischem Material;
• hergestellt aus Kunststoffmaterial;
• vollständig aufblasbar;
• ringförmig;
• rohrförmig;
• zylindrisch geformt;
• konisch geformt;
• fünfeckig geformt.

3. Vorrichtung nach einem der vorangehenden Ansprüche, **dadurch gekennzeichnet, dass** die Auskleidung aus PTFE hergestellt ist.

4. Vorrichtung nach einem der vorangehenden Ansprüche, **dadurch gekennzeichnet, dass** der Stent ausgelegt ist, um innerhalb
• eines Blutgefäßes oder
• von Atemwegen oder
• Harnwegen oder
• Magen-Darm-Passagen oder
• Industrierohren platziert zu werden.

## Revendications

1. Dispositif de commande d'écoulement ou de limitation d'écoulement, comprenant :
une endoprothèse vasculaire destinée à être placée à l'intérieur d'un vaisseau, et
une doublure constituée d'un matériau non réactif fixé à l'intérieur de ladite endoprothèse vasculaire,
**caractérisé en ce que** ladite doublure est gonflable et dégonflable pour commander l'écoulement traversant.

2. Dispositif selon la revendication 1, **caractérisé en ce que** l'endoprothèse vasculaire est conforme à au moins une des possibilités suivantes :
■ réalisée en un matériau métallique,
■ réalisée en une matière plastique,
■ totalement gonflable,
■ mise en forme sous la forme d'un anneau,
■ de forme tubulaire,
■ de forme conique,
■ de forme pentagonale.

3. Dispositif selon l'une quelconque des revendications précédentes, **caractérisé en ce que** la doublure est réalisée en polytétrafluoroéthylène (PTFE).

4. Dispositif selon l'une quelconque des revendications précédentes, **caractérisé en ce que** l'endoprothèse vasculaire est adaptée pour être placée à l'intérieur :
■ d'un vaisseau sanguin, ou
■ des voies aériennes, ou
■ de passages urinaires, ou
■ de passages gastro-intestinaux, ou
■ de tuyaux industriels.
